# EUROPÄISCHE PATENTSCHRIFT

(11) **EP 2 567 614 B1**
(45) Veröffentlichungstag und Bekanntmachung des Hinweises auf die Patenterteilung: **16.09.2015**
(21) Anmeldenummer: 12004925.9
(22) Anmeldetag: 03.07.2012
(51) Int. Cl.: A01K 41/00, A01K 41/02, B01L 1/00, C12M 1/00, C12M 1/02

(54) **Inkubator**
Incubator
Incubateur

(30) Priorität: 12.09.2011 DE 102011112856
(43) Veröffentlichungstag der Anmeldung: 13.03.2013
(73) Patentinhaber: Infors AG, 4103 Bottmingen (CH)
(72) Erfinder: Brücher, Daniel, 40593 Düsseldorf (DE)
(74) Vertreter: Säger, Manfred

(56) Entgegenhaltungen:
- EP-A1- 1 854 871
- DE-A1- 10 344 294
- DE-U1-202007 013 405
- US-A- 5 577 837

## Beschreibung

Die Erfindung betrifft einen gattungsgemässen, als Inkubator, Reaktor od. dgl. ausgebildeten, mittels einer Tür geschlossenen Brutschrank nach dem Oberbegriff des Hauptanspruchs, also einen Inkubator, Reaktor od. dgl. mit einem darin angeordneten, mittels eines Schüttelantriebes angetriebenen, vorzugsweise längs einer Führung aus dem Brutschrank herausfahrbaren Tablar.

Ein solcher gattungsgemässer Brutschrank ist an sich bekannt (EP 1 854 871 A1) und hat sich bewährt. Allerdings weist der bekannte Brutschrank keine Beleuchtungseinrichtung auf, was eine gängige Massnahme darstellt, um in biotechnischen Labors das Zellwachstum von Kulturen zu beeinflussen.

Zu diesem Zweck ist schon eine Beleuchtungseinrichtung bekannt geworden (DE 20 2007 013 405 U1), bei der in einer Halterung für Laborgefässe ein flächiger Träger mit einer Vielzahl von Leuchtdioden (LEDs) so integriert ist, dass diesem der Boden des Laborgefässes zugewandt ist.

Diese bekannte Beleuchtungseinrichtung weist aber mancherlei Nachteile auf. So müssen die LEDs der Beleuchtungseinrichtung auf dem bewegten Schütteltisch montiert werden, wodurch sich Probleme mit dem Stromversorgungskabel ergeben durch die Gefahr der Verletzung des Kabels durch Scheuern und Einklemmen. Auch können die LEDs nur mit Luft gekühlt werden. Eine Kühlung mit Wasser, die eine wesentlich effektivere Ableitung der Abwärme bietet ohne den Inkubator aufzuheizen, ist aber nicht möglich, weil Kühlwasserschläuche nicht am bewegten Schütteltisch montiert werden können. Übedies kann aufgrund der in der Halterung integrierten LEDs nur immer ein Laborgefäss z.B. Schüttelkolben einer bestimmten Größe an einem bestimmten Ort verwendet werden. Der Einsatz von Schüttelkolben unterschiedlicher Grössen und eine abweichende Anordnung z.B. zur besseren Platzausnutzung bei Verwendung von kleineren Schüttelkolben ist ebenfalls nicht möglich. Schliesslich ergibt sich bei der Verwendung von LEDs der Beleuchtungseinrichtung in verschiedenen Farben aufgrund des geringen Abstandes zur Kultur in dem Laborgefäss kein homogenes Lichtspektrum.

Der Erfindung liegt die Aufgabe zugrunde, einen gattungsgemässen Brutschrank für den Einsatz einer Beleuchtungseinrichtung weiterzubilden.

Diese Aufgabe wird erfindungsgemäss bei einem gattungsgemässen Brutschrank sowie Bearbeitungsverfahren nach dem Oberbegriff des Hauptanspruchs erfindungsgemäss durch dessen kennzeichnenende Merkmale gelöst.

Das erfindungsgemässe Prinzip sieht also für den bekannten Schüttelinkubator eine gekühlte Beleuchtungseinrichtung vor, was einen leistungsstärkeren Lichteinsatz gestattet. Die Beleuchtungseinrichtung ist so angeordnet, dass sie sich in kurzem Abstand unter der lichtdurchlässigen oberen Auflageplatte über der -unteren- Platte des somit doppelstöckigen Tablars befindet. Die Laborgefässe mit der darin befindlichen Kultur werden mit dem doppelstöckigem Tablar in den Brutschrankeingeschoben, wobei die untere Platte des Tablars unter die Beleuchtungseinrichtung gleitet und auf einer Kupplungseinrichtung des Schüttelantriebes einrastet. Im Betrieb bewegen sich somit die Laborgefässe mit der darin befindlichen Kultur über der ortsfest stehenden Beleuchtungseinrichtung.

Der erfindungsgemässe Brutschrank zeichnet sich also durch eine stationäre Beleuchtungseinrichtung aus; diese wird nicht mit den Laborgefässen bewegt. Dadurch entfallen Probleme mit der Stromversorgung und es ist die Verwendung eine Kühlung möglich. Ausserdem können die Laborgefässe in unterschiedlichsten Grössen frei auf der oberen Auflageplatte des Tablars platziert werden. Schliesslich ist die Verwendung von Einwegbeuteln möglich.

Dabei ist die obere Auflageplatte mit Distanzstücken versehen, die als doppelstöckiges Tablar mit einer von dieser beabstandet angeordneten unteren Platte unter Bildung eines Zwischenraumes, in den die Beleuchtungseinrichtung hineinragt, oder direkt mit dem Schüttelantrieb verbunden.

Wenn die vorzugsweise an der bezüglich der Tür entgegengesetzt angeordnetete Rückwand des Brutschrankes festgelegte Beleuchtungseinrichtung eine Leiterbahn mit auf deren Abstrahlseite dicht an dicht vorgesehenen LEDs, beispielsweise 8 bis 24 aufweist, kann eine gleichmässige Bestrahlung der gesamten Fläche der oberen Auflageplatte des Tablars erreicht werden.

Ausserdem können Hochleistungs-LEDs eingesetzt werden, wenn die Leiterbahn mit ihrer der Abstrahlseite entgegengesetzten Rückseite an der Kontaktfläche der Kühlung gut wärmeleitend anliegend verbunden ist.

Bei einem Abstandes zwischen LEDs und den Laborgefässen von etwa 5 cm ergibt sich bei Verwendung verschieden farbiger LEDs ein homogenes Lichtspektrum in der Kulturebene der Laborgefässe, wenn jede Leiterbahnen eine Vielzahl von LEDs einer einzigen Farbe aufweist und zumindest zwei Leiterbahnen mit LEDs unterschiedlicher Farbe vorgesehen sind.

Mit Vorteil ist die obere Auflageplatte mit einer z.B. aufgeklebten Haftmatte versehen, woraus eine einfache Herstellung und im Betrieb ein vielfältiger Einsatzvon Laborgefässen aller Art, auch z.B. Einwegbeutel resultiert.

Zweckmässige Ausgestaltungen und Weiterbildungen der Erfindung sind in den restlichen Unteransprüchen gekennzeichnet.

Ein Ausführungsbeispiel der Erfindung wird nachfolgend unter Bezugnahme auf die Zeichnung näher erläutert. In dieser zeigt:
- Figur 1: einen erfindungsgemässsen Brutschrank, in schematischer, perspektivischer Darstellung
- Figur 2: den Brutschrank gemäss Figur 1, im schemati-schen Querschnitt.

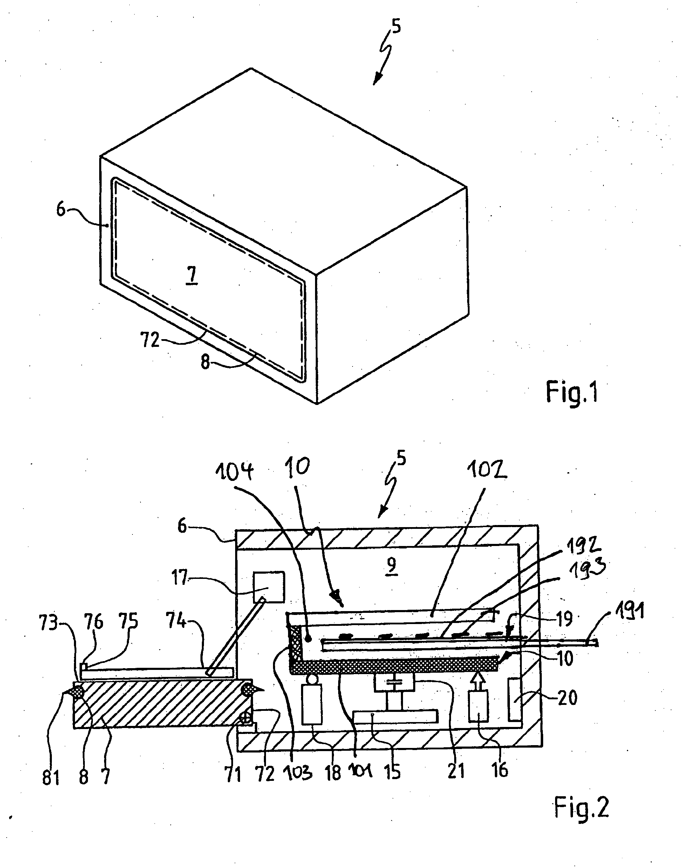

Der als geschlossener Brutschrank 5 ausgebildete Inkubator, Reaktor od. dgl. ist im wesentlichen quaderförmig ausgebildet und weist beim wiedergegbenen Ausführungsbeispiel an seiner Vorderseite 6 eine als um eine horizontale Achse 71 im Bereich ihrer unteren Kante 72 schwenkbare Klappe 7 ausgebildete Tür auf, längs deren Rand eine umlaufende Dichtung 8, wie gestrichelt in Figur 1 angedeutet, mit einer vorspringenden Dichtkante 81 in einer umlaufenden Nut 76 der Klappe 7 angeordnet ist.

Im Inneren 9 des Brutschranks 5, das in Figur 2 gezeigt ist, ist ein Tablar 10 angeordnet, das im Detail noch nachfolgend beschrieben wird. Das Tablar 10 ist über eine Kupplungseinrichtung 21 an den darunter angeordneten Schüttelantrieb 15 angetrieben und kann davon abgekuppelt oder daran angekuppelt, d.h. gelöst werden, so dass das Tablar andere Bewegungsabläufe als den Schüttelbetrieb ausführen kann.

Ferner ist die Klappe 7 mit einer ebenen Innenseite 73 und auf dieser mit einer Führung 74 für das Tablar 10 versehen. Die ebene Innenseite 73 bildet im geöffnetem Zustand der Klappe 7 eine horizontale Fläche, wobei die horizontale Achse 71 der Klappe 7 so angeordnet ist, dass sich die Innenseite 73 der geöffneten Klappe 7 zu der Unterseite des Tablars 10 so fluchtet, dass dieses längs der Führung 74 an der Innenseite 73 der Klappe 7 bis in eine definierte Endstellung mit einer Arretierung 75 herausschiebbar ist, die auch einen ein elektrisches Signal abgebenden Endschalter 76 aufweisen kann.

Das Tablar 10 ist zu dessen Herausschieben aus dem Inneren 9 des Brutschranks 5 sowie zum wieder Hineinziehen dahin längs der Führung 74 ferner mit einem Verschiebeantrieb 18 aus dem Brutschrank 5 bei geöffneter Klappe 7 versehen. Es versteht sich von selbst, dass das Tablar 10, der Schüttelantrieb 15 sowie die Kupplungseinrichtung 21 und die Anhaltepositioniereinrichtung 16 voneinander entkuppelbar sind, so dass es mittels des Verschiebeantriebes 18 längs der Führung 74 aus dem Inneren 9 des Brutschranks 5 herausfahrbar ist. Der Brutschrank 5 sowie die Ablaufsteuerung 20 ermöglichen dabei ein automatisches Verfahren zum Bearbeiten der zu schüttelnden Flüssigkeiten.

Das Tablar 10 ist, wie in Figur 2 gezeigt, mit einer aus einem lichtdurchlässigen Werkstoff bestehenden, vorzugsweise mit einer nicht gezeigten Haftmatte versehene oberen Auflageplatte 102 und einer von dieser über Distanzstücke 103 beabstandet angeordneten, die zu der Tür 7 benachbart angeordnet sind, verbundenen unteren Platte 101 unter Bildung eines Zwischenraumes 104 also doppelstöckig ausgebildet.

In den Zwischenraum 104 ragt eine bezüglich des Brutschrankes 5 ortsfeste, elektrisch betriebene, an der bezüglich der Tür 7 entgegengesetzt angeordneteten Rückwand des Brutschrankes 5 festgelegte Beleuchtungseinrichtung 19 hinein, die mit einer Kühlung 191 gekühlt ist, die über einen Schlauch- oder Rohranschluss aus dem Inneren 9 des Brutschrankes herausgeführt ist. Der Schlauch- oder Rohranschluss der Kühlung 191 ist dabei über die durch die bezüglich der Tür 7 entgegengesetzt angeordnetete Rückwand des Brutschrankes 5 herausgeführt.

Die Beleuchtungseinrichtung 19 weist zumindest zwei Leiterbahnen 192 mit einer auf deren Abstrahlseite vorzugsweise dicht an dicht vorgesehenen Vielzahl von LEDs 193 einer einzigen Farbe auf, wobei die Leiterbahn 192 mit ihrer der Abstrahlseite entgegengesetzten Rückseite an der Kontaktfläche der Kühlung 191 gut wärmeleitend verbunden anliegt. Es sind hierbei zumindest zwei Leiterbahnen 192 mit LEDs unterschiedlicher Farbe vorgesehen.

## Patentansprüche

1. Als Inkubator oder Reaktor ausgebildeter, mittels einer Tür (7) geschlossener Brutschrank (5) mit einem in dessen Inneren (9) angeordneten, mittels eines Schüttelantriebes (15) angetriebenen, vorzugsweise längs einer Führung (74) aus dem Brutschrank (5) herausfahrbaren Tablar (10),
**dadurch gekennzeichnet,**
- **dass** das Tablar (10) mit einer oberen Auflageplatte (102) versehen ist,
- **dass** die Auflageplatte (102) aus einem lichtdurchlässigen Werkstoff besteht,
- **dass** unter die Auflageplatte (102) eine bezüglich des Brutschrankes (5) ortsfeste elektrisch betriebene Beleuchtungseinrichtung (19) hineinragt,
- **dass** diese mit einer Wasser- oder Luft-Kühlung (191) versehen ist und
- **dass** die obere Auflageplatte (102) mit Distanzstücken (103) versehen ist, die als doppelstöckiges Tablar (10) mit einer von dieser beabstandet angeordneten unteren Platte (101) unter Bildung eines Zwischenraumes (104), in den die Beleuchtungseinrichtung hineinragt, oder direkt mit dem Schüttelantrieb (15) verbunden ist.

2. Brutschrank nach Anspruch 1, **dadurch gekennzeichnet, dass** die Kühlung (191) über einen Schlauch- oder Rohranschluss aus dem Inneren (9) des Brutschrankes herausgeführt ist.

3. Brutschrank nach Anspruch 2, **dadurch gekennzeichnet, dass** der Schlauch- oder Rohranschluss der Kühlung (191) über die durch die bezüglich der Tür (7) entgegengesetzt angeordnete Rückwand des Brutschrankes (5) herausgeführt ist.

4. Brutschrank nach Anspruch 1, **dadurch gekennzeichnet, dass** die Beleuchtungseinrichtung (19) an der bezüglich der Tür (7) entgegengesetzt angeordneten Rückwand des Brutschrankes (5) festgelegt ist.

5. Brutschrank nach Anspruch 1 oder 4, **dadurch gekennzeichnet, dass** die Beleuchtungseinrichtung (19) eine Leiterbahn (192) mit auf deren Abstrahlseite vorgesehenen LEDs (193) aufweist.

6. Brutschrank nach Anspruch 5, **dadurch gekennzeichnet, dass** die Leiterbahn (192) mit ihrer der Abstrahlseite entgegengesetzten Rückseite an der Kontaktfläche der Kühlung (191) oder an von der Luft gekühlten Kühlrippen anliegt.

7. Brutschrank nach Anspruch 6, **dadurch gekennzeichnet, dass** jede Leiterbahn (192) eine Vielzahl von LEDs einer einzigen Farbe aufweist.

8. Brutschrank nach Anspruch 7, **dadurch gekennzeichnet, dass** zumindest zwei Leiterbahnen (192) mit LEDs unterschiedlicher Farbe vorgesehen sind.

9. Brutschrank nach Anspruch 1, **dadurch gekennzeichnet, dass** die obere Auflageplatte (102) mit einer Haftmatte versehen ist.

## Claims

1. An incubator (5) formed as an incubator or reactor, which is closed by a door (7), with a shelf (10) arranged in the interior (9) of the incubator, driven by means of a shaking actuator (15), preferably able to be moved out from the incubator (5) along a guide-way (74),
**characterized in**
- **that** the shelf (10) is provided with an upper support plate (102),
- **that** the support plate (102) consists of a light-permeable material,
- **that** an electrically operated lighting device (19), which is fixed with respect to the incubator (5), protrudes under the support plate (102),
- **that** this is provided with a water- or air-cooling system (191) and
- **that** the upper support plate (102) is provided with spacers (103), which is connected as a two-tier shelf (10) with a lower plate (101) arranged spaced apart therefrom with the formation of an intermediate space (104), into which the lighting device protrudes, or directly with the shaking actuator (15).

2. The incubator according to claim 1, **characterized in that** the cooling system (191) is directed out from the interior (9) of the incubator via a hose- or pipe connection.

3. The incubator according to claim 2, **characterized in that** the hose- or pipe connection of the cooling system (191) is directed out via the rear wall of the incubator (5) which is arranged opposite with respect to the door (7).

4. The incubator according to claim 1, **characterized in that** the lighting device (19) is fixed to the rear wall of the incubator (5) which is arranged opposite with respect to the door (7).

5. The incubator according to claim 1 or 4, **characterized in that** the lighting device (19) has a printed circuit board track (192) with LEDs (193) provided on its radiating side.

6. The incubator according to claim 5, **characterized in that** the printed circuit board track (192) lies with its rear side, opposed to the radiating side, against the contact surface of the cooling system (191) or against cooling ribs which are cooled by the air.

7. The incubator according to claim 6, **characterized in that** each printed circuit board track (192) has a plurality of LEDs of a single colour.

8. The incubator according to claim 7, **characterized in that** at least two printed circuit board tracks (192) are provided with LEDs of different colour.

9. The incubator according to claim 1, **characterized in that** the upper support plate (102) is provided with an adhesive mat.

## Revendications

1. Étuve bactériologique (5) conçue en tant qu'incubateur ou réacteur, fermée au moyen d'une porte (7), comprenant une tablette (10) amovible hors de l'étuve bactériologique (5), disposée dans son intérieur (9), entraînée par un entraînement par agitation (15), de préférence le long d'un guide (74),
**caractérisée en ce**
- **que** la tablette (10) est dotée d'une plaque d'appui supérieure (102),
- **que** la plaque d'appui (102) est en matériau translucide,
- **qu'**un dispositif d'éclairage (19) à entraînement électrique, fixe par rapport à l'étuve bactériologique (5), fait saillie sous la plaque d'appui (102),
- **que** celle-ci est dotée d'un refroidissement à eau ou à air (191) et
- **que** la plaque d'appui supérieure (102) est dotée d'écarteurs (103) qui, en tant que tablette à double étage (10) est reliée à un plateau (101) inférieur disposé à distance de celle-ci par formation d'un espace intermédiaire (104) dans lequel le dispositif d'éclairage fait saillie, ou est reliée directement à l'entraînement par agitation (15).

2. Étuve bactériologique selon la revendication 1, **caractérisée en ce que** le refroidissement (191) est sorti de l'intérieur (9) de l'étuve bactériologique par un raccord de tuyau ou de tube.

3. Étuve bactériologique selon la revendication 2, **caractérisée en ce que** le raccord de tuyau ou de tube du refroidissement (191) est sorti par la paroi arrière de l'étuve bactériologique (5) disposée à l'opposé par rapport à la porte (7).

4. Étuve bactériologique selon la revendication 1, **caractérisée en ce que** le dispositif d'éclairage (19) est fixé sur la paroi arrière de l'étuve bactériologique (5) disposée à l'opposé par rapport à la porte (7).

5. Étuve bactériologique selon la revendication 1 ou 4, **caractérisée en ce que** le dispositif d'éclairage (19) présente une piste conductrice (192) avec des LED (193) prévues sur sa face d'émission.

6. Étuve bactériologique selon la revendication 5, **caractérisée en ce que** la piste conductrice (192) repose sur la surface de contact du refroidissement (191) ou sur des nervures de refroidissement refroidies par air, par sa paroi arrière opposée à la face d'émission.

7. Étuve bactériologique selon la revendication 6, **caractérisée en ce que** chaque piste conductrice (192) présente une pluralité de LED d'une seule couleur.

8. Étuve bactériologique selon la revendication 7, **caractérisée en ce qu'**au moins deux pistes conductrices (192) sont dotées de LED de couleur différente.

9. Étuve bactériologique selon la revendication 1, **caractérisée en ce que** la plaque d'appui supérieure (102) est dotée d'un tapis adhérent.
